Europäisches Patentamt

European Patent Office

Office européen des brevets

(19)

(11) Publication number: **0 225 303**
**A1**

(12)

# EUROPEAN PATENT APPLICATION

(21) Application number: **86850402.8**

(22) Date of filing: **24.11.86**

(51) Int. Cl.⁴: **A 61 K 31/19**
**A 61 K 31/20, A 61 K 31/23,**
**A 61 K 9/22**

(30) Priority: **25.11.85 SE 8505569**

(43) Date of publication of application:
**10.06.87 Bulletin 87/24**

(84) Designated Contracting States: **ES GR**

(71) Applicant: **ACO Läkemedel AB**
**Box 3026**
**S-171 03 Solna (SE)**

(72) Inventor: **Bogentoft, Conny**
**Riddersviksvägen 166**
**S-162 40 Vällingby (SE)**

(74) Representative: **Larfeldt, Helene et al**
**Axel Ehmers Patentbyrä AB P.O. Box 5342**
**S-102 46 Stockholm (SE)**

The title of the invention has been amended (Guidelines for Examination in the EPO, A-III, 7.3).

(54) **Use of a hydrophobic substance for the preparation of an enteric preparation for the treatment of obesity, and enteric preparation.**

(57) Use of a hydrophobic substance for preparing an enteric preparation for treatment of obesity. The enteric preparation is an enteric coated capsule, tablet or microcapsules, containing a hydrophobic substance in combination with an emulsifier. In a method for weight reduction, an effective weight reducing dosage of said preparation can be orally administered to a human.

EP 0 225 303 A1

**Description**

ENTERIC PREPARATION

The present invention relates to the use of a hydrophobic substance for preparing an enteric preparation for treatment of obesity. The enteric preparation is a capsule, a tablet or microcapsules coated with a coating resistant to gastric juice which dissolves in ileum, the distsal portion of the small intestine. The invention also relates to a method for weight reduction, wherein said enteric preparation is orally administered in a weight reducing dosage to a human. In a further aspect of the invention also relates to an enteric preparation containing specific hydrophobic substances in combination with an emulsifier.

One of the greatest health problems in modern times is overweight, which is due to the fact that man has not been able to adapt himself to new circumstances requiring less energy but on the whole just as much of certain essential nutrients as before. A heavy overweight, obesity, is a serious medical problem which also leads to many complications.

Overweight is in addition not only a medical problem, but also a matter of well-being and personal satisfaction. It is well known that there is a great demand for effective methods of slimming.

Many drugs having an effect on the weight control in one way or another have been developed. Different investigations have shown that such drugs can bring about a weight loss of about 1-4 kg, but that this weight loss rarely is sustained beyond 8 weeks therapy. These drugs can be of use for therapy during a short period, but hardly offers any long term solution. In the long run fatness apparently can only be treated by changes of the fare and a weight reduction can therefore only be attained by a dietary change (S. Galloway et al, Postgraduate Medical Journal 60, 19-26, 1984).

For most overweight subjects it is hard to effect a weight reduction by slimming, that is a negative energy balance. In spite of different types of special diets it has turned out to be hard to reduce the body weight by means of slimming programs (Läkartidningen 80, no. 50, 4911-15, 1983).

It is generally known that the intake of food is controlled by changes in the composition of nutrients in the blood acting on centers in the hypothalamus, but also by gastrointestinal mechanisms which have still not been explained. It is however known, by means of animal tests, that an extension of the stomach brings about that the animal discontinues to eat, whereas the animal continues to eat for a long time if the food is continuously taken away from the stomach. It has also presently been demonstrated (N.W. Read et al, Gastroenterologi 86. 274-80, 1984) that the presence of unabsorbed food in ileum delays the gastric emptying. By this delay an increased absorption of the ingested food will be attained.

It has now surprisingly turned out that it is possible to effect a reduced food intake by bringing unabsorbed food and especially hydrofobic substances therein into contact with the distal part of the small intestine, that is ileum, where a physiologically mediated mechanism having this effect is started. Tests have shown that ileal infusion of a fat emulsion in connection with a meal brings about that a smaller amount of food is ingested than what should otherwise be the case. This physiologial effect is supposed to depend on that certain types of hydrofobic substances are interacting with specific ileal receptors and thereby induce satiety attended by a reduced food intake.

This finding has been transformed into enteric preparations which are simple and convenient to use for an overweight subject.

The invention relates to the use of a hydrophobic substance for preparing an enteric preparation. In said preparation the hydrophobic substance is used in combination with a emulsifier.

The hydrophobic substance can be a fatty acid having 6-28 carbon atoms, an ester or a salt thereof, a fatty alcohol having 6-28 carbon atoms or an ester thereof.

The fatty acid can be saturated or unsaturated, and have a branched or a straight chain. As examples of fatty acids can be mentioned lauric acid, palmitic acid, stearic acid, oleic acid, ricinoleic acid, linoleic acid and linolenic acid. Preferred acids have 14-22 carbon atoms. Esters of fatty acids can be esters of one or more of said acids and an alcohol containing one or more hydroxyl groups. In a diester one of the ester groups can be derived from a fatty acid having 2-5 carbon atoms. The alcohol can have 2-28 carbon atoms. Examples of esters are isopropyl isostearate, sorbitan trioleate (Span® 85), 1,2-propylene glycol myristate, dipalmitin, acetylated monoglyceride from soybean oil, glyceryl palmitate lactate, hydrogenated tallow glycerides citrate, diacetyl tartaric acid esters of monooleate. A salt refers to a physiologically acceptable salt of an inorganic ion, e.g. sodium. potassium, magnesium or calcium, or an ammonium, mono-, di- or triethanolammonium ion.

The fatty alcohol having 6-28 carbon atoms can be saturated or unsaturated and have a branched or a straight chain. An ester of a fatty alcohol can be an ester of an acid having 1-28 carbon atoms. As an example can be mentioned arachidyl propionate.

As examples of fats. that is mixtures of esters of fatty acids of 6-28 carbon atoms and glycerol, can be mentioned vegetable and animal fats and oils such as borage oil, corn oil, sesame oil, olive oil, soybean oil, cottonseed oil. castor oil. peanut oil. cocoa butter. cod-liver oil. Said mixtures can optionally also contain other substances. such as fatty acids and fatty alcohols.

The hydrophobic substance in the enteric preparation can be in liquid form, such as an oil or a solution or an emulsion of a solid or liquid substance. The hydrophobic substances can also be solid at ambient temperature, provided they are fluid in the intestine.

An emulsifier or dispersing agent can be used tensides and amphoteric compounds which are common within pharmacy, for instance TweenR 80 (polyoxyethylene glycol sorbitan mono-oleat), ArlatonR T (polyoxyethylene fatty acid ester), sodium taurocholate, albumin and lecithin, which are described in Martindale. The Extrapharmacopoeia. 28th ed., 1982. The emulsifier is combined with the hydrophobic substance in an amount of at least 1% by weight based on the hydrophobic substance. The amount to be added should be sufficient to provide a spontaneous emulgation of the hydrophobic substance in the intestine. Free fatty acids generally do not require as much emulsifier as for instance triglycerides of fatty acids. More than 30% should however not be added. a range of 3-15% being preferable.

The enteric prepartion is a capsule, a tablet or microcapsules containing the hydrophobic substance optionally in combination with the emulsifier, which has been coated with a gastric juice resistant coating. The capsules can be hard or soft gelatin capsules containing the active substance in solid and preferably liquid form respectively. Said capsules can also be designed for a slow release of the active substance. Tablets are preferably prepared by moulding the hydrophobic substance, optionally with additives, in accordance with conventional technique. In order to make the preparations unsolvable in acid medium, that is resistant to gastric juice. they are finally coated with a coating of so called gastric juice resistant polymers, which dissolve in the distal portion of the small intestine where the actual receptors are located. An examples of suitable polymers which can be used as coatings can be mentioned cellulose derivatives esterified with phthalic acid, such as cellulose acetate phthalate (CAP), hydroxypropylmethylcellulose phthalate (HP 50, HP 55), and polymers based on methacrylic acid (EudragitR L, Röhm Pharma GmbH) and copolymers based on methacrylic acid methylester (EudragitR S, Röhm Pharma GmbH). These polymers are applied, if required, in admixture with plasticizers and other conventional additives, for instance pigments. It should of course be seen to it that the polymer coating is designed in such a way that it dissolves and releases its content at the intended place in the intestine. Microcapsules can be manufactured by emulsifying the hydrophobic substance in a water solution of a polymer, e.g. cellulose acetate phthalate, and precipitation of the polymer, that is by coacervation, or by spray coating, also in accordance with conventional technique.

The invention also relates to a method for weight reduction. wherein an effective weight reducing dosage of an enteric preparation containing a hydrophobic substance is orally administered to a human. The enteric preparation should be taken in good time before each meal and in order to achieve optimal effect the hydrophobic substance in the preparation should have been brought into contact with the actual receptors in ileum before the meal is started. This means that one or more capsules or tablets or microcapsules containing 1-5g hydrophobic substance and emulsifier should be taken starting 2-5 hours. preferably 2-3 hours before each meal from 1 to 6 times/d. It is of advantage if the preparation is formulated in such a way that the hydrofobic substance is released for a longer period. One dose unit normally comprises 0.01-1 g of the mixture of the hydrophobic substance and the emulsifier.

The invention also relates to an enteric preparation, which is a capsule, a tablet or microcapsules, containing a pharmaceutically active substance and being coated with a gastric juice resistant coating, in which the active substance is a saturated or unsaturated fatty acid having 6-28 carbon atoms, a physiologically acceptable salt thereof, a triglyceride of one or more saturated or unsaturated fatty acids having 6-22 carbon atoms, or a mixture thereof. which is combined with 1-30% by weight based on the hydrophobic substance of an emulsifier.

In a preferred preparation the active substance is a saturated or unsaturated fatty acid having 14-22 carbon atoms, a physiologically acceptable salt thereof or a mixture thereof, which is combined with 3-15% by weight based on the hydrophobic substance of an emulsifier.

The weight reducing effect that is attained by the administration of a hydrophobic substance to ileum will be apparent from the following test.

Infusion of fat emulsion to humans

A fat emulsion comprising 50% corn oil and 3% albumin, and a control solution which only contained 3% albumin were administrated separately at an interval of about a week by ileal infusion to a group of 6 persons in connection with the eating of a appetizing meal.

As a comparison a 20% fat emulsion, essentially containing fractionated soybean oil. Intralipid® (Kabi Vitrum). and a control solution of 0.9% saline were administrated by intravenous infusion to another group of 6 persons under otherwise like conditions.

All the test persons ate until they experienced a feeling of fullness which was scored in the same way on a scale from 1 to 10. The time taken to eat the meal and the quantity of ingested food and drink, as well as the caloric intake calculated therefrom is evident from the table below.

## Table 1

The effect of ileal or intravenous infusion of fat emulsion on food intake

|  | Ileal infusion | | Intravenous infusion | |
|---|---|---|---|---|
|  | Control | Fat emulsion | Control | Fat emulsion |
| Duration of the meal (min) | 31.7±3.0 | 24.8±1.0 | 27.1±2.8 | 25.5±2.6 |
| Amount of food intake (g) | 884±89 | 670±23 | 902±39 | 934±109 |
| Amount of drink intake (ml) | 490±53 | 447.5±46 | 550±62 | 558±61 |
| Total energy intake (kcal) | 1883±259 | 1313±90 | 1965±137 | 1938±236 |

It follows that an ileal infusion of corn oil reduces the amount of food eaten and also the total energy intake. This reduction was greater than the amount of energy which was supplied by the infusion of the fat emulsion. This difference in food intake is not obtained in intravenous infusion of a fat emulsion.

Rat infusion test

The effect of different hydrophobic substances on the gastric emptying was evaluated by means of the following paired study on rats, in which the transit time for an unabsorbable carbohydrate from stomach to caecum was measured by means of hydrogen analysis (see N.J.Brown et al, "Adaption of hydrogen analysis to measure stomach to caesum transit time in the rat". Department of Physiology, The University, Western Bank, Sheffield).

Male albino rats, weighing 250-300 g each, which had been fasted for 18 hours were, after infusion of a test solution or control for 20 minutes through an abdominal sond to the ileum gavaged with 5 ml homogenized baked beans and 0.5 g lactose. The transit of the head of said meal from the stomach to the caecum was assessed by the rise in hydrogen in the environment of the rat, deriving from the fermentation of the unabsorbable carbohydrates in the meal by colon bacteria. The transit time for the test solution and the control were recorded.

The hydrophobic substances to be tested were dispersed in water or emulsified in saline by means of an emulsifier to a concentration of 10-20% by weight. Tween® 80, egg lecithin and/or sodium taurocholate were added to the substances as emulsifiers in an amount of 2-12% by weight based on the hydrophobic substance. The control solution was 0.9% saline to which emulsifier had been added in an amount corresponding to the test solution content. The results of the test are shown in Table 2.

4

## Table 2

Influence of hydrophobic substances on stomach to caecum transit time

| Hydrophobic substance | Concentration % | Transit time * min |
|---|---|---|
| Distilled rapeseed fatty acids **, *** | 20 | 210 |
| Borage oil  ** | 20 | 190 |
| Soybean oil + 10% distilled rapeseed fatty acids **,*** | 20 | 138 |
| Corn oil + 10% distilled rapeseed fatty acids **,*** | 20 | 147 |
| Control | – | 107 |

* average of 2-4 values

** containing 6.3% egg lecithin and 2.5% sodium taurocholate

*** containing 3.5% Tween® 80

It is believed that there is a relation between reduced food intake and delay in gastric emptying.

Example 1

A tablet of the invention can be prepared by moulding a fat which is solid at ambient temperature, such as cocoa butter. in accordance with common technique. Tablets prepared in this way are then coated by means of a coating equipment. For the coating of 5 kg tablets of nonstop type the following composition can for example be used.

| Coating composition | Amount, g |
|---|---|
| polyethylene glycol 6000 | 10.0 g |
| propylene glycol | 39.0 g |
| sorbitan oleat | 13.0 g |
| cellulosaacetate phthalate | 130 g |
| ethanol 70% | 717 g |
| acetone | 1091 g |

After the coating micronised talc is applied 2-5 times in a total amount of 125 g. Tablets coated in this way will obtain a gastric juice resistant coating which is dissolved in ileum.

Example 2

An enteric capsule for oral administration of a hydrophibic substance can be manufactured by loading a soft gelatine capsule (size No. 10 oval. from R.P. Scherer Corporation) with the following mixture.

| Capsule content | % by weight | Amount, mg |
|---|---|---|
| corn oil | 87 | 435 |
| distilled rapeseed fatty acids | 10 | 50 |
| Tween® 80 | 3 | 15 |
| | | in total 500 |

After filling and sealing by means of conventional technique. the capsule was spraycoated with the following solution.

| Coating composition | Approximate amount, mg |
|---|---|
| hydroxypropylmethylcellulose phthalat (HP 55) | 32 |
| dibutylic phthalate | 6.4 |
| ethanol containing 0.75% methylethylketone | 400 |
| water | 70 |

The spraycoating is continued until resistance to gastric juice is obtained (according to USP, Pharm. Eur.). The weight of the coating is then 38.5 mg.

**Claims**

1. Use of a hydrophobic substance for preparing an enteric preparation for treatment of obesity.

2. Use according to claim 1, characterized in that the hydrophobic substance is used in combination with an emulsifier.

3. Use according to claim 1 or 2, characterized in that the enteric preparation is a capsule. a tablet or microcapsules, coated with a gastric juice resistance coating.

4. Use according to any of claims 1-3, characterized in that the hydrophobic substance is a saturated or unsaturated fatty acid having 6-28 carbon atoms, a physiologically acceptable salt thereof or an ester of one or more of said acids and an alcohol containing one or more hydroxyl groups.

5. Use according to any of claims 1-4, characterized in that hydrophobic substance is a glycerol ester of one or more saturated or unsaturated fatty acids having 14-22 carbon atoms or a mixture thereof.

6. Use according to any of claims 1-4, characterized in that that the hydrophobic substance is a saturated or unsaturated fatty acid having 14-22 carbon atoms or physiologically acceptable salt thereof, or a mixture thereof.

7. Method for weight reduction, characterized in orally administering an effective weight reducing dosage of an enteric preparation containing a hydrophobic substance to a human.

8. Method according to claim 7, wherein the enteric preparation is a capsule, a tablet or microcapsules coated with a gastric juice resistant coating, containing 0.01-1 g of a mixture of the hydrophobic substance and an emulsifier. characterized in that 1-5 g of said mixture is administrated 2-3 h before every meal.

9. Enteric preparation. which is a capsule, a tablet or microcapsules containing a pharmaceutically active substance and being coated with a gastric juice resistant coating, characterized in that the active substance is a saturated or unsaturated fatty acid having 6-28 carbon atoms, a physiologically acceptable salt thereof. a triglyceide of one or more saturated or unsaturated fatty acids having 6-22 carbon atoms, or a mixture thereof. which is combined with 1-30% by weight. based on the hydrophobic substance, of an emulsifier.

10. Enteric preparation according to claim 9. characterized in that the active substance is a saturated or unsaturated fatty acid having 14-22 carbon atoms. a physiologically acceptable salt thereof or a mixture thereof. which is combined with 3-15% by weight. based on the hydrophobic substance, of an emulsifier.

European Patent Office

**PARTIAL EUROPEAN SEARCH REPORT**
which under Rule 45 of the European Patent Convention shall be considered, for the purposes of subsequent proceedings, as the European search report

Application number

86850402.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-23 44 958 (ZAHN H) <br> * claims * <br> ----- | 1, 4, 6 | A 61 K 31/19, 31/20, 31/23, 9/22 |
| Y | EP-A-133 110 (SANOFI) <br> * page 2, lines 1-3 and 13-21 and <br> page 7, lines 1-8 <br> claims * <br> ----- | 1, 4 | |
| A | GB-A-2 140 688 (OTSUKA PHARMACEUTICAL) <br> * claims * <br> ----- | | |
| A | GB-A-1 142 804 (LABORATORIES PHARMA SCIENCE) <br> * claims * <br> ----- | | |

TECHNICAL FIELDS SEARCHED (Int. Cl.4)

A 61 K 9/00, 9/22, 9/24, 9/36, 9/42, 9/52, 9/62, 31/19, 31/20, 31/23, 31/25

## INCOMPLETE SEARCH

The Search Division considers that the present European patent application does not comply with the provisions of the European Patent Convention to such an extent that it is not possible to carry out a meaningful search into the state of the art on the basis of some of the claims.
Claims searched completely:
Claims searched incompletely:
Claims not searched: 7-8
Reason for the limitation of the search:

Method for the treatment of the human or animal body.
/Art. 52(4) EPC/

| Place of search | Date of completion of the search | Examiner |
|---|---|---|
| STOCKHOLM | 11-02-1987 | TANNERFELDT A. |

EPO Form 1505.1 03.82